# FASCICULE DE BREVET EUROPEEN

(11) **EP 3 675 886 B1**
(45) Date de publication et mention de la délivrance du brevet: **14.05.2025**
(21) Numéro de dépôt: 18807885.1
(22) Date de dépôt: 08.11.2018
(51) Int. Cl.: A61K 35/742, A61K 35/747, A61P 31/04, A61K 9/00

(54) **COMPOSITION PROBIOTIQUE DE BACILLUS ET DE BACTÉRIES LACTIQUES POUR AMÉLIORER LA SANTÉ DES ANIMAUX D'ÉLEVAGE**
PROBIOTISCHE ZUSAMMENSETZUNG ENTHALTEND BACILLUS UND MILCHSÄUREBAKTERIEN ZUR VERBESSERUNG DER GESUNDHEIT VON VIEH
PROBIOTIC COMPOSITION COMPRISING BACILLUS AND LACTIC ACID BACTERIA FOR IMPROVING THE HEALTH OF LIVESTOCK

(30) Priorité: 08.11.2017 FR 1760496
(43) Date de publication de la demande: 08.07.2020
(73) Titulaire: MIXSCIENCE, 35170 Bruz (FR)
(72) Inventeur: LAYUS, Michel, 22560 Pleumeur Bodou (FR); BRAME, Alexandre, 35000 Rennes (FR)
(74) Mandataire: LLR
(86) Numéro de dépôt international: PCT/EP2018/080670
(87) Numéro de publication internationale: WO 2019/092136

(56) Documents cités:
- WO-A1-2013/178947
- WO-A1-2014/151837
- DATABASE WPI Week 201543, Derwent World Patents Index; AN 2015-241592, XP002783772
- MANEEWAN C ET AL: "Development of Bacillus subtilis MP and effective utilization on productivity and microorganisms in feces of suckling piglets", INTERNATIONAL JOURNAL OF APPLIED RESEARCH IN VETERINARY MEDICINE, VETERINARY SOLUTIONS, US, vol. 9, no. 4, 1 January 2011 (2011-01-01), pages 382 - 387, XP002688379, ISSN: 1559-4602
- LEE D J ET AL: "Evaluation of probiotic treatment in a neonatal animal model.", PEDIATRIC SURGERY INTERNATIONAL 2000, vol. 16, no. 4, 2000, pages 237 - 242, XP055499223, ISSN: 0179-0358

## Description

L'invention concerne une composition pour améliorer la santé des animaux, notamment des mammifères d'élevage.

Les mammifères naissent avec un tube digestif stérile. L'apparition de la flore intestinale dépend donc, essentiellement, de l'environnement et des particularités physiologiques de chaque espèce. Dans ces conditions, c'est le colostrum ingéré pendant les premiers jours de vie qui protègera en principe le nouveau-né contre les infections digestives. Cette protection n'est possible que si le colostrum maternel est lui-même qualitativement et quantitativement adapté et approprié.

La protection immunitaire mammaire prend pour point de départ un processus immunitaire intestinal ce qui impacte inévitablement la mise en place de cette immunité. Il en résulte l'apparition possible d'infections néonatales chez le jeune mammifère dès la 24ème ou 48ème heure de vie, en raison d'une protection insuffisante et ce jusqu'au 21ème jour, date d'apparition de l'immunité locale.

L'industrie des aliments pour animaux est de plus en plus considérée comme ayant un rôle à jouer pour assurer l'utilisation responsable des antimicrobiens alimentaires dans la production animale. Les décideurs demandent aux producteurs d'aliments pour animaux, aux agriculteurs, aux vétérinaires et aux organismes de réglementation de travailler ensemble pour déterminer les meilleures pratiques d'élevage et d'hygiène et des alternatives viables, afin de réduire l'utilisation d'antibiotiques chez les animaux d'élevage et d'améliorer le bien-être des animaux.

Pour répondre aux nouvelles exigences de l'agriculture moderne, et tenant compte des contraintes biologiques des mammifères, il est nécessaire de mettre au point une méthode de protection de l'environnement d'élevage et de la flore intestinale des nouveau-nés.

L'art antérieur propose des applications sur les mamelles des femelles dans le but de traiter les mammites, par exemple la demande RU2612009. Toutefois, ces méthodes ont pour objectif de traiter les mamelles des femelles, mais ne visent pas à améliorer la santé des nouveau-nés. La demande WO 2013/178947 divulgue l'utilisation de Bacillus subtilis, notamment les souches NOLO1, NOL02, NOL03 ou NOLO4, et de lactococcus lactis, notamment la souche NOL11, pour le traitement des infections dans les élevages aviaires. En revanche, le traitement de pathologies des mammifères n'est pas divulgué par ce document.

D'autres méthodes connues de l'état de la technique visent quant à elles à traiter l'environnement d'élevage des nouveau-nés afin de favoriser leur croissance et leur bien-être.

Malgré ces tentatives, le besoin de proposer une amélioration des conditions favorable au bon développement des nouveau-nés demeure.

L'invention vise à remédier à ce manque.

Un objet de l'invention est de proposer une méthode de prévention des risques sanitaires et d'amélioration de la performance de l'élevage de mammifères, ainsi qu'une composition microbienne.

Aussi, l'invention concerne une composition consistant essentiellement en un mélange :
a. de trois souches de bactéries du genre *Bacillus subtilis,* et
b. d' une souche de bactérie lactique, pour son utilisation dans le cadre de la prévention ou le traitement des infections contractées par un nouveau-né de mammifères d'élevage au moment de la naissance, notamment les infections digestives ou respiratoires,

ladite composition étant appliquée, sur le périnée et/ou sur la ou les mamelles de la femelle allant donner ou ayant donné naissance audit nouveau-né de mammifères d'élevage, l'application étant réalisée avant et/ou après la mise-bas,
ledit mélange consistant essentiellement en
   - les trois souches de *Bacillus subtilis* suivantes : NOL01, NOL02, NOL03, lesdites souches étant déposées à la CNCM sous les numéros respectifs :CNCM I - 4606, CNCM I-5043 et CNCM I - 4607, et
   - la souche de bactérie lactique : *Lactococcus lactis spp lactis* 1 souche NOL11, ladite souche étant déposée à la CNCM sous le numéro CNCM I - 4609.

L'invention repose sur la constatation surprenante faite par les inventeurs que l'utilisation combinée d'au moins une souche de bactérie lactique et au moins une souche de *Bacillus,* lorsqu'elles sont appliquées, avant ou après la mise bas, sur le périnée et/ou la ou les mamelles de femelles permettent de protéger, dès le plus jeune âge (dès la mise bas), les nouveau-nés contre les infections néfastes à leur développement, et ainsi favoriser et améliorer leur croissance.

La composition telle qu'utilisée dans l'invention présente l'avantage d'avoir une action interne à l'animal et dans son environnement extérieur, permettant de créer une flore de barrière dès le plus jeune âge.

Dans l'invention, par « mamelles et/ou périnée », on entend que l'application se fait soit sur le périnée seul, soit sur une ou plusieurs mamelles seules, soit en combinant des applications sur le périnée et au moins sur une mamelle, voire toutes les mamelles.

Avantageusement, dans l'invention une application sur le périnée peut couvrir soit une application sur le périnée seul, soit une application intra vaginale, soit une application à la fois sur le périnée et intra vaginale.

Avantageusement, dans l'invention, une application sur la ou les mamelles correspond à une application externe réalisée sur les mamelles des femelles mammifères, mais peut aussi correspondre à une application intra mammaire desdites mamelles desdites femelles de mammifères.

Les applications intra mammaires sont réalisées au moyen, par exemple, de seringues introduites dans le conduit des trayons des mamelles desdites femelles de mammifères. L'homme du métier connaît les techniques les plus appropriées pour réaliser de telles applications intra mammaires.

Dans l'invention il est important que ce soit la femelle qui met bas le nouveau-né à traiter qui reçoive l'application de la composition susmentionnée. En effet, l'intérêt est que le nouveau dès qu'il est expulsé de l'utérus de la femelle soit en contact avec le périnée traité, et/ou que ses premières tétées se fassent sur une mamelle traitée.

Par « au moins une souche de bactérie du genre *Bacillus* » on entend qu'une souche, ou deux souches, ou trois souches ou plus de *Bacillus* peuvent être utilisées dans le contexte de l'invention. De la même manière, par « au moins une souche de bactérie lactique », on entend qu'une souche, ou deux souches, ou trois souches ou plus, peuvent être utilisées dans la composition définie ci-dessus.

Par souche de bactéries, on entend dans l'invention l'ensemble des individus (bactéries) issus de repiquages successifs d'une colonie bactérienne.

En d'autres termes, une souche est une partie d'une espèce bactérienne différente des autres bactéries de la même espèce par une différence mineure mais identifiable. Une souche est également définie comme une population de bactéries qui descend d'un seul organisme ou de la culture isolat pur. Les souches d'une même espèce peuvent différer légèrement de l'autre à bien des égards.

L'application sur le périnée et/ou la ou les mamelles est réalisée dans des conditions où la totalité ou la quasi-totalité des parties traitées soient recouvertes par la composition selon l'invention. En effet, il n'est pas forcément nécessaire que la partie traitée soit complètement recouverte, l'essentiel est que la quantité de composition soit suffisante pour que le nouveau-né soit en contact avec suffisamment de bactéries pour le protéger contre les infections.

L'application de la composition selon l'invention étant réalisée avant et/ou après la mise bas, cela signifie que
- la composition est appliquée sur le périnée, avant, après ou avant et après la mise bas,
- la composition est appliquée sur la ou les mamelles, avant, après ou avant et après la mise bas, ou encore
- la composition est appliquée sur le périnée et la ou les mamelles, avant, après ou avant et après la mise bas.

Les applications mamelles et/ou périnée peuvent être réalisées simultanément, séparément ou étalées dans le temps.

En dehors du cadre de l'invention il est décrit une composition pour l'utilisation susmentionnée, dans laquelle la composition bactérienne comprend :
- au moins l'une des trois souches de *Bacillus subtilis* suivantes : NOL01, NOL02, NOL03, lesdites souches étant déposées à la CNCM sous les numéros respectifs : CNCM I - 4606, CNCM I-5043 et CNCM I - 4607, et
- au moins la souche de bactérie lactique: *Lactococcus lactis spp lactis* 1 souche NOL11, ladite souche étant déposée à la CNCM sous le numéro CNCM I - 4609.

En dehors du cadre de l'invention il est décrit combinaisons suivantes :
- NOL01 et NOL11,
- NOL02 et NOL11,
- NOL03 et NOL11,
- NOL01, NOL02 et NOL11,
- NOL01, NOL03 et NOL11,
- NOL02, NOL03 et NOL11.

Toutes ces souches ont été déposées à la collection nationale des microorganismes (CNCM) à l'Institut Pasteur à Paris, selon le traité de Budapest.

Plus avantageusement, l'invention concerne la composition pour son utilisation susmentionnée, ladite composition comprenant de 10⁴ à 10¹¹ colonies bactériennes de *Bacillus* et de 10⁴ à 10¹¹ colonies bactériennes de bactérie lactique, les colonies bactériennes étant par mL ou g de composition.

En d'autres termes, dans ce mode de réalisation avantageux, si la composition selon l'invention est sous forme liquide, ladite composition comprendra de 10⁴ à 10¹¹ colonies bactériennes de *Bacillus* par mL de composition et de 10⁴ à 10¹¹ colonies bactériennes de bactérie lactique par mL de composition.

Si par contre la composition est sous forme non aqueuse, voire déshydratée, ladite composition comprendra de 10⁴ à 10¹¹ colonies bactériennes de *Bacillus* par g de composition et de 10⁴ à 10¹¹ colonies bactériennes de bactérie lactique par g de composition.

Dans l'invention, de 10⁴ à 10¹¹ colonies bactériennes signifie : environ 10⁴, environ 5. 10⁴, environ 10⁵, environ 5.10⁵, environ 10⁶, environ 5.10⁶, environ 10⁷, environ 5.10⁷, environ 10⁸, environ 5.10⁸, environ 10⁹, environ 5.10⁹, environ 10¹⁰, environ 5.10¹⁰ ou environ 10¹¹ colonies bactériennes.

Une application particulière pour la femelle mammifère est de 10⁵ à 10¹¹ colonies bactériennes par femelle, pour chacune des applications. C'est-à-dire que chaque bactérie sera présente de 10⁵ à 10¹¹ colonies bactériennes par femelle pour l'application périnée, et de 10⁵ à 10¹¹ colonies bactériennes par femelles pour l'application mamelle.

L'homme du métier sait aisément comment déterminer ce nombre de bactéries, notamment par comptage soit manuel (en utilisant une lame de Malassez) ou en utilisant un compteur de cellule automatique, ou par dilution puis ensemencement sur gélose et comptage des colonies.

Encore plus avantageusement, l'invention concerne l'utilisation telle que définie précédemment, où au moins une souche de *Bacillus* est sous forme sporulée et/ou sous forme végétative.

Les bactéries lactiques sont quant à elles toujours sous forme végétatives. Aussi la composition selon l'invention comprend au moins une souche de *Bacillus* sous forme sporulée et au moins une souche de bactérie lactique sous forme végétative, ou comprend au moins une souche de *Bacillus* sous forme végétative et au moins une souche de bactérie lactique sous forme végétative.

De manière avantageuse, l'invention concerne une composition pour son utilisation telle que définie ci-dessus, ladite composition étant appliquée, sur le périnée et/ou sur la ou les mamelles de la femelle allant donner ou ayant donné naissance audit nouveau-né de mammifères d'élevage, l'application étant réalisée avant et/ou après la mise bas, ladite femelle étant dans un environnement préalablement traité avec ladite composition.

Dans l'invention, il est avantageux qu'avant l'application sur la ou les mamelles et/ou le périnée des femelles, l'environnement d'élevage desdites femelles soit traité avec la composition selon l'invention avant que lesdites femelles soient introduites dans ledit environnement ou le jour même de leur arrivée.

L'application dans l'environnement permet de contrôler et/ou d'orienter la flore bactérienne de l'environnement des femelles en diminuant la pression pathogène. Elle peut se faire en nébulisation ou pulvérisation des bâtiments d'élevage, incorporation à la litière... On observe moins de contamination des animaux car il y a moins de foyers pathogènes. Une telle application joue sur les pathogènes vivants ou survivants dans l'environnement. Les modes d'épandage dans l'environnement sont la pulvérisation, la nébulisation, le recouvrement des surfaces par des mousses ou encore le saupoudrage de poudre de composition selon l'invention.

L'invention concerne la composition pour l'utilisation susmentionnée, ladite composition consistant essentiellement en
- les trois souches de *Bacillus subtilis* suivantes : NOL01, NOL02, NOL03, lesdites souches étant déposées à la CNCM sous les numéros respectifs : CNCM I - 4606, CNCM I-5043 et CNCM I - 4607, et
- la souche de bactérie lactique: *Lactococcus lactis spp lactis* 1 souche NOL11, ladite souche étant déposée à la CNCM sous le numéro CNCM I - 4609.

Il est particulièrement avantageux que la composition comprenne les trois souches de bactéries *Bacillus subtilis* susmentionnées, sous forme végétative ou sporulée, et la souche de bactérie lactique susmentionnée, sous forme végétative.

De manière encore plus avantageuse, l'invention concerne la composition susmentionnée pour son application susmentionnée, où lesdites infections sont des infections intestinales notamment les diarrhées néonatales, les entérites, la salmonellose, des infections respiratoires et en particulier la rhinite, la pasteurellose, la bordetellose, les infections cutanées et en particulier les ulcères, les épidermites, les nécroses, et les infections au niveau du système locomoteur notamment les boiteries infectieuses, notamment les différentes formes d'arthrite.

En effet, la flore que constitue la composition selon l'invention, comme illustré dans les exemples ci-dessous, sera ingérée par les nouveau-nés et permettra de contrôler et/ou d'orienter la flore présente dans les systèmes digestifs et/ou respiratoires en diminuant la pression pathogène existante ou entrante (les systèmes digestif et respiratoire étant des voies d'entrée possibles de pathogènes responsables de pathologies liées à d'autres systèmes de l'organisme). Un autre effet sera la stimulation des défenses naturelles des animaux. La flore joue également directement sur la santé digestive des animaux et limite les entrées d'autres pathogènes, ce qui est avantageux tant que le nouveau-né n'a pas acquis son immunité définitive.

De manière avantageuse, l'invention concerne l'utilisation susmentionnée, où ladite composition est appliquée sur le périnée et/ou la ou les mamelles par pulvérisation, étalement, saupoudrage ou trempage.

L'homme du métier, selon le mode d'application utilisé, saura choisir le matériel le plus approprié, et notamment préfèrera une composition liquide pour la pulvérisation ou le trempage et favorisera une composition déshydratée pour une application par saupoudrage.

Un kit de prévention des infections digestives, respiratoires ou autres, des nouveau-nés de mammifères d'élevage comprenant essentiellement :
- au moins une composition telle que définie précédemment, et
- au moins un moyen d'application de ladite composition sur le périnée et/ou la ou les mamelles de la femelle ayant donné ou allant donner naissance audit nouveau-né de mammifères d'élevage,
est également décrit.

Le kit peut se composer d'un mélange prédéfini de bactéries tel que défini ci-dessus, ou de plusieurs flacons ou sachets séparés comprenant une ou plusieurs souches bactériennes selon l'invention.

Les moyens d'applications contenus dans le kit susmentionné peuvent être des pinceaux pour une application par étalement, des cartouches à utiliser pour une pulvérisation, un pulvérisateur, un outil de trempage ou tout autre moyen permettant une application telle que définie ci-dessus notamment la pulvérisation, l'étalement, le saupoudrage ou le trempage.

L'invention sera mieux comprise et illustrée à la lecture des exemples et des figures suivantes :

### BREVE DESCRIPTION DES DESSINS

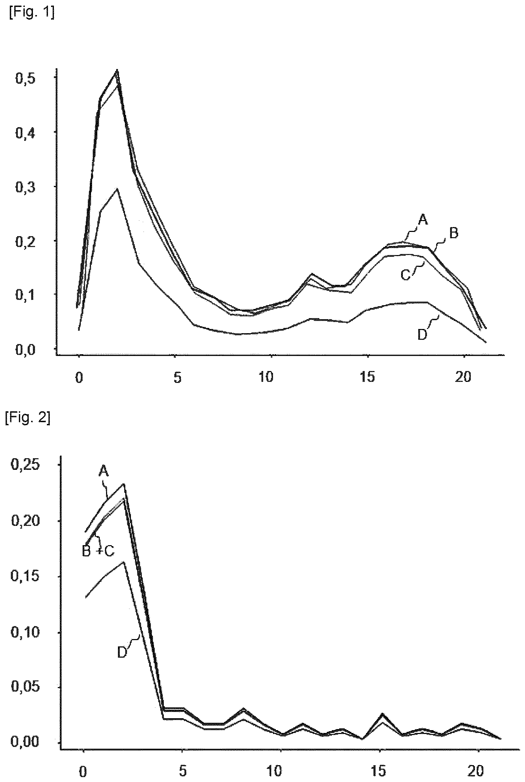

[Fig.1] est un graphique montrant la probabilité de survenue d'une diarrhée chez les porcelets dans une case, en fonction du temps (en jours) après la naissance (t=0), sur les lots 1 à 3 (courbes A à C) traités avec un antibiotique, et le lot 4, traité seulement avec la composition selon l'invention (courbe D).
[Fig.2] est un graphique montrant la probabilité de mortalité (à l'exclusion de la mortalité éliminés « petits ») chez les porcelets dans une case, en fonction du temps (en jours) après la naissance (t=0), sur les lots 1 à 3 (courbes A à C) traités avec un antibiotique, et le lot 4, traité seulement avec la composition selon l'invention (courbe D).

### EXEMPLES

### Exemple 1

L'objectif de cet essai est de réduire l'utilisation des antibiotiques en élevage. Cette volonté s'accompagne d'un fort investissement de développement de toutes les solutions alternatives : vaccination, conduite d'élevage, biosécurité et produits de substitution... Agir sur les écosystèmes bactériens qui interagissent avec l'animal est donc une voie prometteuse et l'utilisation de flores de barrière s'inscrit dans cette démarche.

Cet essai montre les résultats obtenus chez un éleveur déjà utilisateur de flores de barrière en péri mise-bas et qui souhaitait réévaluer son protocole (une nouvelle formulation de la flore de barrière (NOL01, NOL02, NOL03, NOL 11) ainsi qu'un rythme d'application allégé).

### Matériel et méthodes

L'élevage suivi est un élevage de 700 truies naisseur engraisseur en sélection multiplication (5 bandes de 120 truies). L'essai s'est déroulé sur 2 bandes à l'intérieur desquelles 3 lots de 40 truies ont été constitués en respectant une répartition homogène des rangs de portée. Le protocole d'application comprend la pulvérisation de la composition selon l'invention dans l'environnement, sur les mamelles et le périnée des truies ainsi qu'une administration probiotique aux truies via la machine à soupe.

Le tableau 1 résume les protocoles de pulvérisation appliqués. L'ensemble des truies a reçu une dose de probiotiques à l'auge de J-3 à J1 par rapport à la mise bas.

Tableau 1 : Protocoles de pulvérisation des flores

**[Table 1]**

| | **Lot 1 - témoin** | **Lot 2 - témoin** | **Lot 3- témoin** | **Lot 4 essai** |
|---|---|---|---|---|
| **Rythme d'application** | Ancien | Ancien | Nouveau allégé | Nouveau allégé |
| **Formule** | Ancienne | Nouvelle | Nouvelle | Nouvelle |
| **Bande** | 1 + 2 | 1 | 1 + 2 | 2 |
| **Antibiotique sur porcelets** | oui | oui | oui | **non** |
| **J-3** | Environnement | Environnement | Environnement | Environnement |
| **J-2** | | | | |
| **J-1** | Mamelles et périnée | Mamelles et périnée | Mamelles et périnée | Mamelles et périnée |
| **J0 : Mise bas** | | | | |
| **J1** | Mamelles et périnée | Mamelles et périnée | Mamelles et périnée | Mamelles et périnée |
| **J4** | Mamelles et périnée | Mamelles et périnée | | |

Pour les lots 1 à 3, l'éleveur a réalisé une injection d'antibiotique aux porcelets au moment des soins il s'agit donc des lots témoins ; il a accepté de suspendre cette administration sur une partie des porcelets correspondant au lot 4, il s'agit donc du lot test. En effet, l'antibiotique a un effet sur la composition selon l'invention et annule donc son effet potentiel. Les lots 1 à 3 représentent donc les lots témoins positifs (traités aux antibiotiques) et le lot 4, le lot essai, représente une application sur les mamelles et le périnée.

On réalise ensuite un enregistrement quotidien sur les critères suivants :
- Notation des fèces : **0** Normales, **1** Molles (dégradé), **2** Bouses, **3** Liquides,
- Propreté des porcelets : **0** souillés <20%, **1** souillés entre 20 et 50%, **2** souillés à plus de 50%
- mortalité et causes
- traitements et motifs.

Les analyses statistiques ont été réalisées sous le logiciel R (logiciel de référence en libre accès ; R Development Core Team (2008). R: A language and environment for
statistical computing. R Foundation for Statistical Computing, Vienna, Austria). Le modèle utilisé est le modèle logistique.

### Résultats

### 1) Diarrhées des porcelets

Dans un premier temps, juste après la naissance, l'émergence de diarrhée chez au moins un porcelet par case est évaluée. Les résultats sont montrés à la Figure 1.

La modélisation statistique montre que le risque d'apparition de diarrhées chez le nouveau-né, dans une case, est donc significativement réduit chez les porcelets ayant été en présence de la composition selon l'invention appliquée sur le périnée et les mamelles de leur mère, comparé aux mêmes porcelets traités avec un antibiotique.

Le risque est ici réduit de 50%.

On notera que dans le cadre du lot 4, l'éleveur a réalisé 30% de traitements médicamenteux en moins pour soigner les animaux.

### 2) Mortalité des porcelets

Différentes causes de mortalité ont été identifiées : éliminés « petits » (autour de 7% des porcelets), mortalité pour cause digestive (environ 2%), mortalité par écrasement (environ 2%) et autres causes (environ 4%).

Pour l'analyse des résultats, les inventeurs n'ont pas retenu la cause « éliminés petits » considérant qu'elle n'est pas impactée par l'application de la composition selon l'invention en péri mise bas. La modélisation représente le risque de mortalité toutes autres causes confondues à l'échelle d'une case (Figure 2).

L'évolution du risque de mortalité du lot 4 est significativement différente des 3 autres lots (p-value =0.067).

### Discussion

Cette étude montre une équivalence de résultats entre les deux formulations et rythmes d'application des flores de barrière.

Des différences positives significatives ont été mises en évidence avec le protocole sans injection d'antibiotique aux porcelets sur l'apparition de diarrhées et la mortalité globale. Cette amélioration est liée à l'absence d'inhibition potentielle de l'antibiotique sur les flores.

### Conclusion

Les observations faites sur le lot sans antibiotique montrent clairement l'efficacité sur la santé des porcelets (moins de diarrhées et de mortalité) de la composition selon l'invention lorsque le porcelet est en contact immédiatement à la naissance avec la composition selon l'invention.

### Exemple 2

Cet essai a pour but de confirmer l'intérêt de l'application de flores (NOL01, NOL02, NOL03, NOL 11) en maternité directement sur les mamelles et périnée des truies dans un objectif d'amélioration de la santé des porcelets, et notamment la réduction des diarrhées néonatales.

### 1- Protocole

L'essai a eu lieu dans une maternité collective porcine, sur 2 bandes consécutives, avec pour chaque bande, une salle témoin et une salle essai d'environ 18 places chacune. L'essai s'est déroulé de l'entrée des truies en maternité jusqu'au sevrage des porcelets à 21 jours.

### a-Planning d'essai et applications :

Avant le lancement de l'essai, un cycle de nettoyage-désinfection de 2 jours a été réalisé dans toutes les salles, témoins et essais, avant l'arrivée des truies en maternité.

A l'arrivée des truies en maternité à J-3, J0 étant le jour de la 1ère mise-bas, l'environnement d'élevage, à savoir l'ensemble des parois, sols et matériels (cloisons, nourrisseurs, ...) des salles essais a été traité par pulvérisation de la solution selon l'invention (NOL01, NOL02, NOL03, NOL 11).

A partir de J-1, une application de la solution selon l'invention telle que préparée selon le protocole b- ci-dessous a été réalisée par pulvérisation sur les mamelles et périnée des truies essais. Cette double application mamelles et périnée a ensuite été renouvelée de façon journalière jusqu'à la mise-bas.

Après mise-bas, seule l'application sur mamelles a été renouvelée tous les jours jusqu'à J5 dans les salles essais.

Dans les premiers jours de sa vie et ce, jusqu'au sevrage, le porcelet se nourrit exclusivement du lait maternel (aucun autre aliment, ni liquide ni solide). Le porcelet va donc téter régulièrement les mamelles de sa mère. Ainsi, une application de la solution selon l'invention sur les mamelles des mères permet l'ingestion des flores par les porcelets.

Après mise-bas, les porcelets et l'état des cases ont été surveillés jusqu'au sevrage, soit 3 semaines (21 jours).

### b- Mise en oeuvre de la solution selon l'invention :

Une dose de la solution selon l'invention est conditionnée dans deux flacons distincts de 20 ml chacun :
- Un flacon B contenant des Bacillus (NOL01, NOL02 et NOL03)
- Un flacon L contenant les Lactococcus (NOL11)

Les flacons sont apportés congelés en élevage.

Préparation d'une solution mère :
Une solution mère a été préparée après décongelation d'une dose de la solution selon l'invention (1 flacon B et un flacon L) pendant 1h à température ambiante ou 5 min dans de l'eau froide. Cette dose décongelée a été ajoutée à 310 mL d'eau potable non traitée dans un bidon doseur auquel on fait subir une homogénéisation par agitation. La solution mère ainsi préparée a été conservée au réfrigérateur jusqu'à la fin des applications (J5).

### Préparation pour pulvérisation :

Chaque jour de l'essai (de J-1 à J5), 50 mL de la solution mère ont été prélevés et versés dans le réservoir d'un pulvérisateur n'ayant pas contenu de désinfectant, auxquels sont ajoutés 500 mL d'eau potable non traitée. Cette nouvelle préparation a ensuite été pulvérisée sur les mamelles et le périnée des truies des salles essai selon le protocole a- décrit ci-dessus.

### 2- Mesures

Les paramètres zootechniques suivants ont été relevés pendant toute la durée de la maternité, jusqu'au sevrage des porcelets à 21 jours, dans chacune des cases essai et témoin :
- Croissance : poids individuel des porcelets (relevé hebdomadaire pendant toute la période de maternité, soit 3 fois) ;
- Diarrhées : nombre de cases avec présence de diarrhées et proportion estimée de porcelets touchés par case ;
- Mortalité digestive : nombre de porcelets morts par case du fait d'une pathologie digestive ;
- Antibiotiques : nombre de traitements antibiotiques appliqués par case, et nature du traitement.

### 3- Résultats

Une validation a posteriori de la mise en lot a été réalisée par l'analyse des poids des porcelets à la naissance, du nombre de porcelets par truie et par lot, de la date de la mise-bas et du rang de portée.

**[Table 2]**

| | **Bande 1** | | **Bande 2** | |
|---|---|---|---|---|
| **Lot** | **Essai** | **Témoin** | **Essai** | **Témoin** |
| Diarrhées (% du nombre de cases touchées) | 60%^{a} | 85%^{b} | 85% | 85% |
| Traitements antibiotiques (nombre par case) | 0,6^{a} | 2,4^{b} | 1,9^{a} | 3,3^{b} |
| Mortalité digestive (nombre de morts par case) | 0,5 | 0,8 | 0,7^{a} | 2,0^{b} |
| Poids individuel (en kg, à 17j) | 4,58^{a} | 4,01^{b} | 4,02 | 4,20 |

| | | | | |
|---|---|---|---|---|
| a, b : différences statistiquement significatives intra-bandes (Anova III, p<0,05) | | | | |

### Observations :

Sur la bande 1, on observe significativement moins de diarrhées néonatales dans le lot essai par rapport au lot témoin. De même pour le nombre de traitements antibiotiques par case sur les 21 jours d'essai et d'observation. Le poids moyen des porcelets est supérieur de +570g pour les porcelets du lot essai par rapport au lot témoin, cette différence étant statistiquement significative.

Sur la bande 2, malgré un pourcentage identique de cases touchées par des diarrhées entre le lot essai et le lot témoin, on note une diminution du nombre de traitements antibiotiques chez le lot essai et une baisse de la mortalité liée aux troubles digestifs, toutes deux statistiquement significatives. Cela signifie que même si les animaux essais ou témoin de la bande 2 présentent le même pourcentage de diarrhées, on note un avantage chez les animaux ayant reçu la composition selon l'invention (les diarrhées sont moins violentes).

### 4- Conclusions

Les résultats de cet essai permettent de confirmer l'intérêt d'une application de la solution selon l'invention sur les mamelles et le périnée des truies par rapport à l'absence d'application : ainsi, si on favorise l'ensemencement interne (via ingestion par succion des mamelles) et externe (via contact avec le périnée de la mère) de flores positives chez le porcelet dès la mise-bas, on améliore la santé de ces jeunes animaux, et notamment la santé digestive ce qui se traduit par une réduction significative du nombre de traitements antibiotiques et/ou des diarrhées néonatales, voire de la mortalité digestive.

### 5- Observations post-essai

L'éleveur impliqué dans cet essai a souhaité poursuivre les applications sur les 8 bandes suivantes (bandes 3 à 10).

Le même protocole a été suivi, mais uniquement avec des salles essais. Les résultats sont très satisfaisants et il a été observé une réduction des diarrhées néonatales significatives selon l'éleveur.

Cette poursuite de l'essai a conduit l'éleveur à modifier sa gestion des pathologies des jeunes porcelets, notamment en arrêtant les traitements antibiotiques systématiques, et en limitant l'utilisation de produits tels que les asséchants.

## Revendications

1. Composition consistant essentiellement en un mélange :
a- de trois souches de bactéries du genre *Bacillus subtilis,* et
b- d' une souche de bactérie lactique,
pour son utilisation dans la prévention ou le traitement des infections contractées par un nouveau-né de mammifères d'élevage au moment de la naissance, notamment les infections digestives ou respiratoires,
ladite composition étant appliquée, sur le périnée et/ou sur la ou les mamelles de la femelle allant donner ou ayant donné naissance audit nouveau-né de mammifères d'élevage, l'application étant réalisée avant et/ou après la mise-bas, ledit mélange consistant essentiellement en
- les trois souches de *Bacillus subtilis* suivantes : NOL01, NOL02, NOL03, lesdites souches étant déposées à la CNCM sous les numéros respectifs :CNCM I - 4606, CNCM I-5043 et CNCM I - 4607, et
- la souche de bactérie lactique : *Lactococcus lactis spp lactis* 1 souche NOL11, ladite souche étant déposée à la CNCM sous le numéro CNCM I - 4609

2. Composition pour son utilisation selon la revendication 1, ladite composition comprenant de 10⁴ à 10¹¹ colonies bactériennes de *Bacillus* et de 10⁴ à 10¹¹ colonies bactériennes de bactérie lactique, les colonies bactériennes étant par mL ou g de composition.

3. Composition pour son utilisation selon l'une quelconque des revendications 1 à 2, où au moins une souche de *Bacillus* est sous forme sporulée et/ou sous forme végétative.

4. Composition pour son utilisation selon l'une quelconque des revendications 1 à 3, ladite composition étant appliquée, sur le périnée et/ou sur la ou les mamelles de la femelle allant donner ou ayant donné naissance audit nouveau-né de mammifères d'élevage, l'application étant réalisée avant et/ou après la mise bas, ladite femelle étant dans un environnement préalablement traité avec ladite composition.

5. Composition pour son utilisation selon l'une quelconque des revendications 1 à 4, où lesdites infections sont des infections intestinales, des infections respiratoires et en particulier la rhinite, la pasteurellose, la bordetellose, les infections cutanées et en particulier les ulcères, les épidermites, les nécroses, et les infections au niveau du système locomoteur.

6. Composition pour son utilisation selon la revendication 5, où les infections au niveau du système locomoteur sont des boiteries infectieuses.

7. Composition pour son utilisation selon la revendication 5 où les infections intestinales sont les diarrhées néonatales, les entérites ou la salmonellose.

8. Composition pour son utilisation selon l'une quelconque des revendications 1 à 7, où ladite composition est appliquée sur le périnée et/ou la ou les mamelles par pulvérisation, étalement, saupoudrage ou trempage.

## Patentansprüche

1. Zusammensetzung, im Wesentlichen bestehend aus einer Mischung:
a- von drei Bakterienstämmen der Gattung
Bacillus subtilis, und
b- einem Milchsäurebakterienstamm,
zur Verwendung bei der Vorbeugung oder Behandlung von Infektionen, die sich ein Neugeborenes von Nutzsäugetieren bei der Geburt zugezogen hat, insbesondere Infektionen des Verdauungstrakts oder der Atemwege,
wobei die Zusammensetzung auf das Perineum und/oder das/die Gesäuge des weiblichen Nutzsäugetiers aufgetragen wird, das kurz vor der Geburt steht oder das Neugeborene geboren hat, und die Auftragung vor und/oder nach der Geburt erfolgt, wobei die Mischung im Wesentlichen besteht aus
- den drei folgenden Stämmen von *Bacillus subtilis:* NOL01, NOL02, NOL03, wobei die Stämme bei der CNCM unter den jeweiligen Nummern CNCM I-4606, CNCM I-5043 und CNCM I-4607 hinterlegt sind, und
- dem Milchsäurebakterienstamm: *Lactococcus lactis spp. lactis 1* Stamm NOL11, wobei der Stamm bei der CNCM unter der Nummer CNCM I-4609 hinterlegt ist.

2. Zusammensetzung zur Verwendung nach Anspruch 1, wobei die Zusammensetzung 10⁴ bis 10¹¹ Bakterienkolonien von *Bacillus* und 10⁴ bis 10¹¹ Bakterienkolonien von Milchsäurebakterien umfasst, wobei die Bakterienkolonien pro ml oder g der Zusammensetzung angegeben sind.

3. Zusammensetzung zur Verwendung nach einem der Ansprüche 1 bis 2, wobei mindestens ein Stamm von *Bacillus* in sporulierter Form und/oder in vegetativer Form vorliegt.

4. Zusammensetzung zur Verwendung nach einem der Ansprüche 1 bis 3, wobei die Zusammensetzung auf das Perineum und/oder das/die Gesäuge des weiblichen Nutzsäugetiers aufgetragen wird, das kurz vor der Geburt steht oder das Neugeborene geboren hat, und die Auftragung vor und/oder nach der Geburt erfolgt, wobei sich das Weibchen in einer Umgebung befindet, die zuvor mit der Zusammensetzung behandelt wurde.

5. Zusammensetzung zur Verwendung nach einem der Ansprüche 1 bis 4, wobei es sich bei den Infektionen um Darminfektionen, Infektionen der Atemwege und insbesondere Rhinitis, Pasteurellose, Bordetellose, Hautinfektionen und insbesondere Geschwüre, Epidermitis, Nekrose und Infektionen des Bewegungsapparates handelt.

6. Zusammensetzung zur Verwendung nach Anspruch 5, wobei es sich bei den Infektionen des Bewegungsapparats um infektiöse Lahmheit handelt.

7. Zusammensetzung zur Verwendung nach Anspruch 5, wobei es sich bei den Darminfektionen um neonatale Diarrhöe, Enteritis oder Salmonellose handelt.

8. Zusammensetzung zur Verwendung nach einem der Ansprüche 1 bis 7, wobei die Zusammensetzung durch Sprühen, Verteilen, Bestäuben oder Eintauchen auf das Perineum und/oder das/die Gesäuge aufgetragen wird.

## Claims

1. Composition consisting essentially of a mixture:
a- of three strains of bacteria of the genius *Bacillus subtilis,* and
b- a strain of lactic bacteria,
for its use for the prevention or treatment of infections contracted by a newborn livestock mammal at the time of birth, in particular digestive or respiratory infections said composition being applied to the perineum and/or to the teat(s) of the female that is going to give birth or has given birth to said newborn livestock mammal, the application being carried out before and/or after parturition,
said mixture consisting essentially of
- the following three *Bacillus subtilis* strains: NOL01, NOL02, NOL03, said strains being deposited in the CNCM under the numbers: CNCM I - 4606, CNCM I-5043 and CNCM I - 4607, respectively, and
- the lactic bacteria strain: *Lactococcus lactis spp lactis* 1 strain NOL11, said strain being deposited in the CNCM under number CNCM I - 4609.

2. Composition for the use thereof according to claim 1, said composition comprising from 10⁴ to 10¹¹ bacterial colonies of Bacillus and from 10⁴ to 10¹¹ bacterial colonies of lactic bacteria, the bacterial colonies being per mL or g of composition.

3. Composition for the use thereof according to anyone of claims 1 to 2, wherein at least one *Bacillus* strain is in sporulated and/or vegetative form.

4. Composition for the use thereof according to anyone of claims 1 to 3, said composition being applied to the perineum and/or to the teats of the female that is going to give birth or has given birth to said newborn livestock mammals, the application being carried out before and/or after parturition, said female being in an environment previously treated with said composition.

5. Composition for the use thereof according to any of claims 1 to 4, wherein said infections are intestinal infections, in particular neonatal diarrhea, enteritis, salmonella, respiratory infections, and in particular rhinitis, pasteurellosis, bordetellosis, cutaneous infections, and in particular ulcers, epidermitis, necroses and infections in the locomotive system, in particular infectious lameness.

6. Composition for the use thereof according to claim 5, wherein infections in the locomotive system are infectious lameness.

7. Composition for the use thereof according to claim 5, wherein intestinal infections are neonatal diarrhea, enteritis or salmonella.

8. Composition for the use thereof according to any of claims 1 to 7, wherein said composition is applied to the perineum and/or the teat(s) by spraying, spreading, powdering or soaking.
